# EUROPEAN PATENT APPLICATION

(11) **EP 2 383 260 A2**
(43) Date of publication of application: **02.11.2011**
(21) Application number: 11162992.9
(22) Date of filing: 19.04.2011
(51) Int. Cl.: C07D 215/14

(54) **Process for the preparation of statins**

(30) Priority: 30.04.2010 IT MI20100753; 14.03.2011 IT MI20110397
(71) Applicant: Dipharma Francis S.r.l., 20021 Baranzate (MI) (IT)
(72) Inventor: TADDEI, Maurizio, 20021, BARANZATE (MI) (IT); BALDUCCI, Evita, 20021, BARANZATE (MI) (IT); ATTOLINO, Emanuele, 20021, BARANZATE (MI) (IT); MANTEGAZZA, Simone, 20021, BARANZATE (MI) (IT); Dell'anna Gianmaria, 20021 Baranzate (MI) (IT)
(74) Representative: Minoja, Fabrizio

(57) **Abstract**

Process for the preparation of ß-ketoester synthetic intermediates useful in the preparation of statins, in particular Pitavastatin.

## Description

### FIELD OF THE INVENTION

The present invention relates to a novel process for the preparation of synthetic intermediates with ß-ketoester structure useful in the preparation of statins, in particular Pitavastatin.

### TECHNOLOGICAL BACKGROUND

Statins are a class of pharmaceuticals useful in the treatment of cholesterolemia. Their action mechanism is related to their ability to act as inhibitors of 3-hydroxy-3-methylglutaric coenzyme A reductase (HMG-CoA) activity, hence inhibiting the cholesterol biosynthesis in the liver. The molecular structure of statins usually consists of a portion A and a side chain as depicted below 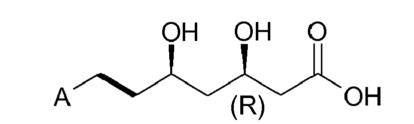 wherein the bond in bold 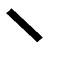 is a single 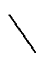 or double 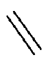 bond.

Statins activity is indeed ascribed to the presence of this side chain, which substantially consists of a 3,5-dihydroxy heptanoic acid residue which mimics the 3-hydroxy-3-methylglutaric moiety of HMG-CoA.

The (R) configuration at C-3 and the corresponding *syn* configuration between the hydroxyls has proved essential to obtain a high level of inhibitory activity. In all the statins developed and marketed to date, the main chemical problem to be solved has been the synthesis of the optically active residue of 3,5-dihydroxy heptanoic acid. The problem has so far been solved using a wide variety of methodologies, such as enzymatic and microbiological methods (see Angew. Chem. Int. Ed. 2005, 44, 362-365) and synthesis methods (Helvetica Chimica Acta, 2007, 1069-1081). The synthesis methods currently available include some processes involving enantioselective synthesis of the acid, and others leading to the formation and subsequent resolution of the racemate. All these methodologies have produced optically active synthesis intermediates, now commercially available, which, when inserted into the structure of the statin, lead to synthesis of the side chain in optically active form.

In the case of synthesis methods which involve resolution of the racemate, or enzymatic methods which do not lead to derivatives of sufficiently high optical purity for the pharmacopoeia specifications, treatments that increase the value of the enantiomeric excess and the chemical purity in general (such as purifications with chiral HPLC or crystallisation of diastereomeric salts) are needed to obtain the required statin with enantiomeric excess values exceeding 99.5%.

The first statin was mevastatin, isolated from *Penicillium citrinum*, which was subsequently used to synthesise the semisynthetic statin pravastatin. The statins now on the market, such as semisynthetic Pravastatin and Lovastatin of completely natural origin, are characterised by having the side chain bonded to the A nucleus with a C-C or C-N single bond (the bond shown in bold in the formula reported above). Recently, however, statins of completely synthetic origin such as Rosuvastatin, Fluvastatin and Pitavastatin, which possess very high biological activity, have become widespread on the market. Said statins are characterised by a side chain bonded with a C=C double bond with strictly (E) geometry (bond shown in bold in the formula reported above) to the A nucleus of the statin, which normally has a complex aromatic structure. Once again, the approaches to solving the problem of the absolute configuration of the double bond were many and varied, and the proposed syntheses were both consecutive and convergent. In particular, however, methods of obtaining convergent formation of the statin with a double bond with (E) geometry have been developed and optimised. These methods are based on the classic methodologies of double (E) bond formation with olefination reactions on suitable aldehydes, which can be present on either the aromatic portion or the side chain of the statin, by means of Wittig, Horner-Emmons or Julia reactions, as clearly summarised in Organic Process Research & Development 2001, 5, 519-527 for Fluvastatin.

Although these methods have produced various statins which have achieved great commercial success, said syntheses still suffer in many cases from problems of regioselectivity, associated with the formation of the statin impurity with Z double bond, cost problems associated with the use of expensive reagents and organic bases, which are delicate or have to be prepared in situ under sophisticated reaction conditions, and in the case of phosphoranes and phosphonates, used for the Wittig reaction, problems of disposal of the wastewater containing organic phosphorus.

There is consequently a need for a more advantageous alternative method of preparing statins, especially those with formula (**I**) shown here, containing a -C=C- double bond with an (E) configuration between the side chain and nucleus **A**, and intermediates useful for the synthesis thereof. Said new method should in particular be more simply industrially scalable, preferably involve the use of more stable, cheaper reagents, and at the same time provide high yields and process wastewater which is more environmentally compatible and easily disposed of.

### SUMMARY OF THE INVENTION

It has now surprisingly been found that statins of formula (**I**) containing a double bond -C=C- with (E) configuration, can be prepared from intermediates of formula (**II**), obtained by Knoevenagel condensation. The resulting intermediates of formula (**II**) have a content in isomer with configuration (Z) lower than 1%, as evaluated by means of HPLC.

### DETAILED DISCLOSURE OF THE INVENTION

Object of the present invention is a process for the preparation of a compound of formula (**II**), or a salt thereof, 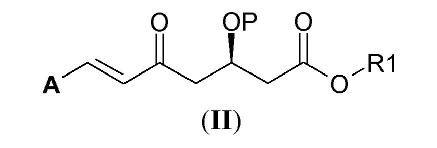 wherein **A** is a statin residue, P is a protective group, and R1 is hydrogen, an optionally substituted C1-C12 alkyl group, cycloalkyl, aryl, or an optionally substituted aryl-C1-C12 alkyl group;
comprising the condensation of an aldehyde of formula **(III)**

**A**―CHO (III)

wherein **A** is as defined above, with a ß-ketoacid compound of formula (**IV**), or a salt thereof, 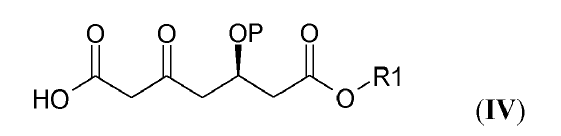 wherein P and R1 are as defined above, in the presence of a catalyst, and if necessary in a solvent, and the subsequent spontaneous decarboxylation.

By way of example, **A** can be the residue of a statin selected from Pitavastatin, Fluvastatin, Glenvastatin, Rosuvastatin, Cerivastatin and Bervastatin. Said residue can be represented by one of the cyclic structures reported below. 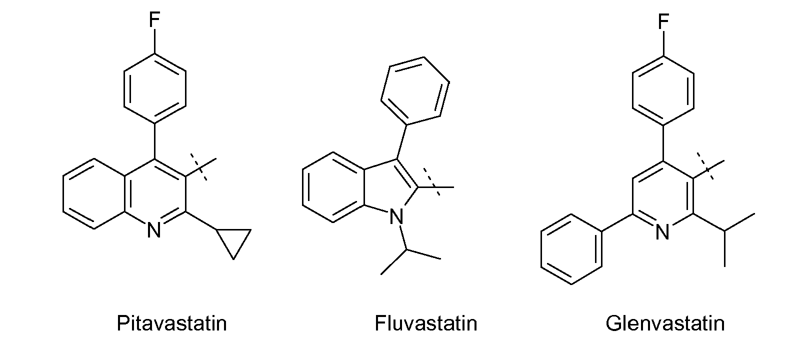 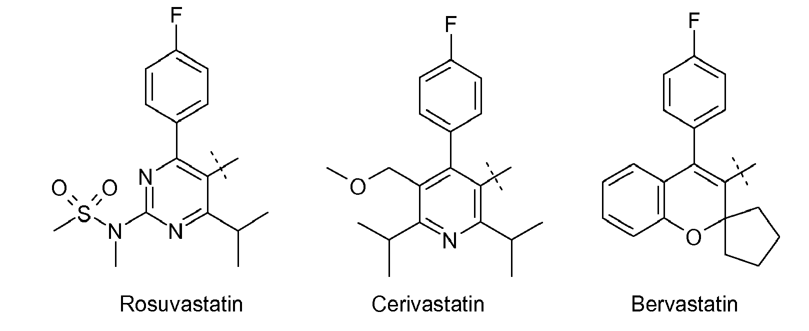 wherein the dotted line indicates the site at which the side chain is attached to the statin residue. Preferably the residue **A** is the nucleus of Pitavastatin, Fluvastatin or Rosuvastatin.

**P** is one of the protective groups used in sugars chemistry, preferably the tert-butyl dimethylsilyl group.

A C1-C12 alkyl group can be straight or branched, unsubstituted or substituted with one or two substituents independently selected from hydroxy, acetoxy and C1-C4 alkoxy, preferably a C1-C4 alkyl group, more preferably methyl or tert-butyl.

A cycloalkyl group can be for example a C3-C8 cycloalkyl group, for example cyclohexyl.

An aryl group is for example a C6-C12 aryl group, preferably phenyl or naphthyl, in particular phenyl.

An aryl-C6-C12 alkyl group, wherein the alkyl is unsubstituted or substituted with one or two substituents independently selected from hydroxy, acetoxy and C1-C4 alkoxy, and the aryl is for example benzyl or phenylethyl, preferably benzyl.

The Knoevenagel condensation between a compound of formula (III) and a compound of formula (IV) can be carried out according to known methods, in particular in the presence of a catalyst.

More particularly, the catalyst can be a basic or acid, organic or inorganic catalyst or an amino acid catalyst.

An organic basic catalyst can be selected from a secondary amine and a tertiary amine, either weak or strong, cyclic or acyclic, or a salt thereof, for example piperidine or a salt thereof, typically the acetate salt.

An inorganic basic catalyst can be selected from e.g. a carbonate or a hydroxide of an alkali metal, preferably sodium or potassium.

Alternatively the Knoevenagel condensation can be carried out with a Lewis acid catalyst. A Lewis acid can be selected for example from ZnCl2, FeC13, TiC14, Ti tetraisopropoxide, A1C13, BF3 etherate, a halide, e.g. a chloride, or a trifluoromethanesulfonate of a transition metal of the lanthanides series, preferably lanthanum trifluoromethanesulfonate in both the anhydrous and hydrated forms.

An amino acid can be, for example, an α-amino acid, ß-amino acid, γ-amino acid, δ-amino acid, or a ε-amino acid; in the case of an α-amino acid, it can bear a residue of natural or synthetic origin in the side chain. Said amino acid can be selected from e.g. the group comprising glycine, α-alanine, valine, leucine, isoleucine, serine, threonine, phenylalanine, tyrosine, proline, aspartic acid, asparagine, glutamic acid, L,α-aminoadipic acid, ß-alanine, γ-aminobutyric acid, δ-aminovaleric acid, ε-aminocapronic acid, taurine and L-phenylserine. The amino acid is preferably selected from the group consisting of glycine, α-alanine, ß-alanine, δ-aminovaleric acid and ε-aminocapronic acid; more preferably ß-alanine.

The Knoevenagel condensation, if necessary, can be carried out in a solvent, for example a polar aprotic solvent, typically an amide, e.g. dimethylformamide, dimethylacetamide or N-methylpyrrolidone, preferably dimethylacetamide; acetonitrile; dimethylsulfoxide; or in a solvent selected from an ether, e.g. tetrahydrofuran or dioxane; a chlorinated solvent, e.g. dichloromethane, dichloroethane, chloroform or chlorobenzene; an ester, e.g. ethyl or methyl acetate; an apolar aprotic solvent typically toluene; or a polar protic solvent, e.g. water or a C1-C5 alkanol, preferably methanol; or a mixture of two or more, preferably of two or three, of said solvents.

The reaction can be carried out at a temperature ranging from about 0°C to the reflux temperature of the solvent, preferably from about 25°C to 85°C, more preferably from 35°C to 45°C.

The resulting crude compound of formula (**II**) has the double bond in (E) configuration and a content in isomer with (Z) configuration lower than 1%, evaluated by HPLC.

A further object of the invention is a statin of formula (**I**), or a salt thereof, with a content in isomer with (Z) configuration lower than 1%, preferably ranging from about 0.01 to 0.1%, evaluated by HPLC.

A thus obtained compound of formula (**II**), or a salt thereof, can be converted to a statin of formula (**I**), or a salt thereof, according to known procedures, for example according to the following scheme reported in WO 03064392. 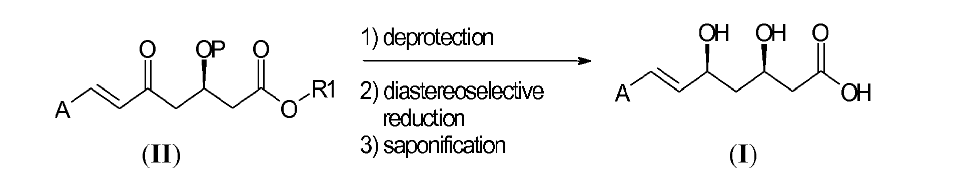 wherein A, P and R1 are as defined above.

Therefore the invention further provides a process comprising the conversion of a compound of formula (**II**), or a salt thereof, obtained starting from a compound of formula (**IV**), to a statin of formula (**I**), or a salt thereof, 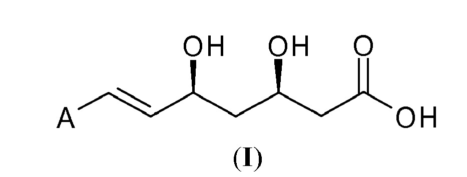 wherein **A** is a statin residue.

In a preferred aspect of the invention, a salt of a compound of formula (**I**), wherein A is the Pitavastatin residue, is a salt with (R)-naphthylethylamine [(R)-NEA], having the following formula (**IX**)

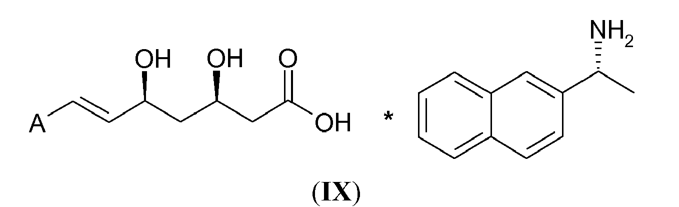

It has indeed surprisingly been found that the Pitavastatin salt with (R)-naphthylethyl amine, of formula (**IX**), can be isolated by crystallization, for example from an organic solvent, preferably a ketone, typically acetone, an alkanol, typically isopropanol, or a mixture thereof or a mixture of one or two of them with water, to obtain a product with both chemical and optical high purity level, which is in both cases equal to or higher than 99%, typically equal to or higher than 99.5%.

More particularly, Pitavastatin salt with (R)-naphthylethyl amine of formula (**IX**) having such a high purity can be converted to another Pitavastatin salt, for example the calcium salt, with known procedures.

A Pitavastatin salt thus obtained using such a highly pure salt of formula (**IX**), has in its turn both chemical and optical high purity level, which is in both cases equal to or higher than 99%, typically equal to or higher than 99.5%.

Said conversion can be carried out, for example, by treatment of an aqueous solution of the salt of formula (**IX**) with an acid, followed by extraction with an organic solvent, e.g. a carboxylic acid alkyl ester, in particular ethyl acetate, or an ether, in particular t-butyl methyl ether, to obtain a Pitavastatin solution, which can optionally be converted to another pharmaceutically acceptable salt, e.g. the calcium salt.

An acid can be for example an aqueous solution of an organic or mineral acid, preferably an HCl aqueous solution.

A further object of the invention is therefore a process for the purification of Pitavastatin, or a salt thereof, comprising the conversion of Pitavastatin or a salt thereof, to a Pitavastatin salt with (R)-naphthylethyl amine, the isolation in the crystalline form, and the subsequent conversion to Pitavastatin or another salt thereof.

The Pitavastatin salt with (R)-naphthylethyl amine of formula (**IX**), in particular in the solid form, preferably in the crystalline form, is a novel compound and is a further object of the invention.

The compounds of formula **(III)** can be prepared as described for example in Organic Process Research & Development 2001, 5, 519-527, for the case of Fluvastatin, and are commercially available.

A compound of formula (**IV**), or a salt thereof, can be prepared starting from a compound of formula (**V**), or a salt thereof, by selective cleavage of the ester group R2, 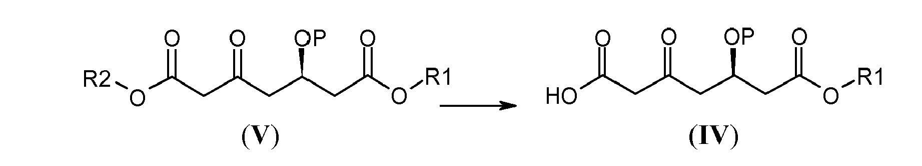 wherein P is as defined above; R2 is a C1-C12 alkyl, cycloalkyl, aryl, or aryl-C1-C12 alkyl group; and R1 is as defined above. In a compound of formula (**V**), when R1 is different from hydrogen, R1 and R2 can be the same or different. Preferably R2 is benzyl and R1 is methyl.

Preferably R2 can be removed to give the free carboxylic acid of formula (**IV**) selectively with respect to the **P** protection and optionally to the group R1 when this is different from hydrogen. By way of example, according to a preferred aspect of the invention, when in a compound of formula (**V**) R1 is methyl and R2 is benzyl, the benzyl group can be cleaved by catalytic hydrogenation with known methods.

The compounds of formula (**IV**) and (**V**), or a salt thereof, are novel and are a further object of the present invention.

A further object of the invention is the use of a compound of formula (**IV**) in a process for the preparation of a statin of formula (**I**).

A compound of formula (**V**), or a salt thereof, can be prepared by reaction between a compound of formula (**VI**), or a salt thereof, and a compound of formula (**VII**), or a salt thereof, in the presence of a solvent 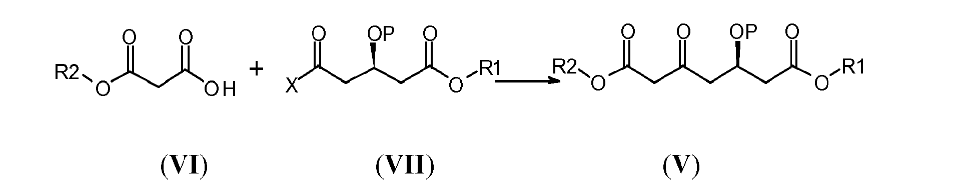 wherein P, R1 and R2 are as defined above and X is a leaving group, preferably a halogen, in particular chlorine, or imidazole. The solvent is preferably an ether solvent, e.g. tetrahydrofuran or an apolar aprotic solvent, typically toluene.

The malonic acid monoester (**VI**) wherein R2, being as defined above, is preferably benzyl, can be first converted to its magnesium salt by treatment with at least 2 equivalents of a Grignard reagent, for example isopropylmagnesium chloride, then reacted with a compound of formula (**VII**), to provide, upon spontaneous decarboxylation, the compound of formula (**V**).

A compound of formula (**VI**) is commercially available or can be prepared by mono esterification of malonic acid with known procedures.

A compound of formula (**VII**), or a salt thereof, can in its turn be prepared by activation of the carboxylic function of a compound of formula (**VIII**), or a salt thereof, 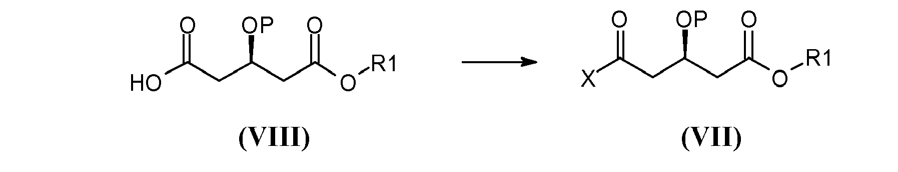 wherein P, R1 and X are as defined above.

The activation of the carboxylic acid of formula (**VIII**) can be attained using for example thionyl chloride, when X is chlorine, or carbonyldiimidazole, when X is imidazole.

A compound of formula (**VIII**) is commercially available or can be prepared for example by chemical desymmetrization of hydroxyglutaric anhydride protected with the protective group P, defined above, as reported for example in J. Org. Chem. 1994, 59, 7849-7854, or by enzymatic desymmetrization, as disclosed in Angew. Chem. Int. and. 2005, 44, 362-365.

A salt of a compound of formula (**II**), (**IV**), (**V**), (**VI**), (**VII**) or (**VIII**) is for example a pharmaceutically acceptable salt.

If desired, a compound of formula (**I**), (**II**), (IV), (**V**), (**VI**), (**VII**) or (**VIII**) can be converted to a salt thereof, or a salt of a compound of formula (**I**), (**II**), (**IV**), (**V**), (**VI**), (**VII**) or (**VIII**) can be converted to the free acid, according to known methods.

The following examples illustrate the invention.

### Example 1. Synthesis of a compound of formula (VII): (R)-methyl 3-(tert-butyldimethylsilyloxy)-5-(1H-imidazol-1-yl)-5-oxopentanoate

A solution of (S)-benzyl mandelate (6 g, 24.79 mmol) in anhydrous THF (100 mL) under N2 atmosphere is cooled to -78°C and a solution of BuLi 2.5 M in hexane (10.9 mL, 27.27 mmol) is added therein drop by drop. The mixture is kept under stirring at -78°C for 20 minutes, then a solution of 3-[(tert-butyldimethylsilyl)oxy]pentanedioic anhydride (6.05 g, 24.79 mmol) in anhydrous THF (25 mL) is added and the resulting mixture is kept under magnetic stirring at -78°C for 2 hours. The reaction is acidified with 1 N HCl and extracted with AcOEt. The organic phase is washed with 1 N HCl and brine, dried over Na2SO4, filtered and evaporated under reduced pressure. The reaction crude is purified by flash silica gel chromatography (eluent: EtPet/AcOEt 4:1) and the resulting product is dissolved in anhydrous THF (150 ml), then treated with dimethyl dicarbonate (2.47 mL, 23.04 mmol) and DMAP (201 mg, 1.65 mmol). The mixture is kept under stirring at 25°C for 20 minutes, then the solvent is evaporated off under reduced pressure and the residue purified by flash chromatography (eluent: EtPet/AcOEt 9:1). The colourless oil obtained is dissolved in ethyl acetate (90 mL) and the resulting solution is kept under stirring under H2 atmosphere in the presence of 20% Pd(OH)2/C (652 mg, 0.93 mmol) at 20°C for 10 hours. The mixture is filtered through perlite and the solvent is evaporated off under reduced pressure. The reaction crude (containing phenylacetic acid as impurity) is dissolved in anhydrous THF under N2 atmosphere and treated with 1,1'-carbonyldiimidazole (3.02 g, 18.68 mmol). The mixture is left under stirring at 20°C for 3 hours. The solvent is evaporated off under reduced pressure and the residue is purified by flash silica gel chromatography (eluent: EtPet/AcOEt 30:70). 2.85 g of a pale yellow oil are obtained (yield 41.6% over 4 steps).

¹H NMR (400 MHz, CDCl3) δ: 8.06 (s, 1H); 7.37 (s, 1H), 6.92 (s, 1H); 4.54-5.52 (m, 1H); 3.53 (s, 3H); 3.04-2.96 (s, 2H); 2.50-2.40 (m, 2H); 0.62 (s, 9H); -0.1 (s, 3H), -0.21 (s, 3H). ¹³C NMR (100 MHz, CDCl3) δ: 170.36; 167.19; 136.06; 130.47; 115.68; 65.69; 51.11; 42.35; 41.41; 24.95; 17.17; -5.58; -5.79. mS(ES⁺): m/z 349 [M+Na]⁺.

### Example 2. Synthesis of a compound of formula (V): (R)-1-benzyl 7-methyl 5-(tert-butyldimethylsilyloxy)-3-oxoheptanedioate

Isopropyl magnesium chloride (2 M in THF, 15.10 mL, 30.20 mmol) is added drop by drop to a solution of monobenzyl malonate (2.93 g, 15.10 mmol) in anhydrous THF (28 mL) at 0°C under N2 atmosphere. After 30 minutes at 0°C the solution is heated at 50°C for 30 minutes, then cooled again to 0°C and a solution of (**VII**) prepared as in Example 1 (4.1 g, 12.58 mmol) in anhydrous THF (28 mL) is slowly added thereto. The mixture is left under stirring at 20°C for 12 hours, then added with 1M HCl and extracted with Et₂O. The organic phase is washed with 1M HCl and brine, dried over Na2SO4, filtered and concentrated under reduced pressure. The reaction crude is purified by flash silica gel chromatography (eluent: EtPet/AcOEt 9:1). 3.74 g of a yellow oil are obtained in 73% yield.

¹H NMR (400 MHz, CDCl3) δ: 7.32-7.28 (m, 5H); 5.12 (s, 2H); 4.57-4.52 (m, 1H); 3.60 (s, 3H); 3.47 (s, 2H); 2.75 (d, J = 6, 2H); 2.51-2.37 (m, 2H); 0.81 (s, 9H); 0.03 (d, J = 10. 3H). ¹³C NMR (100 MHz, CDCl3) δ: 200.23; 170.76; 166.24; 134.93; 128.14; 127.95; 127.83; 127.66; 66.61; 65.06; 51.04; 49.89; 49.54; 41.65; 25.26; 17.42; -5.29; -5.45. MS(ES⁺): m/z 431 [M+Na]⁺.

### Example 3. Synthesis of a compound of formula (VII): (R)-methyl 3-(tert-butyldimethylsilyloxy)-5-(1H-imidazol-1-yl)-5-oxopentanoate

A solution of (R)-benzyl mandelate (49.4 g, 204 mmol) in anhydrous THF (500 mL) under N2 atmosphere is cooled to -78°C and a 2.3 M hexyllithium solution in THF (97 mL, 224 mmol, 1.1 equivalents) is added drop by drop. The mixture is kept under stirring at -78°C for 30 minutes, then a solution of 3-[(tert-butyldimethylsilyl)oxy]pentanedioic anhydride (50.0 g, 204 mmol) in anhydrous THF (100 mL) is added at -78°C and the resulting mixture is kept under magnetic stirring at -78°C for 2 hours. The reaction is warmed to -15°C, acidified with 1 N HCl and extracted with AcOEt. The organic phase is washed with 1 N HCl and brine, dried over Na2SO4, filtered and evaporated under reduced pressure. The reaction crude is dissolved in cyclohexane (250 ml) and kept under stirring at 20°C for 16h. The precipitated solid is filtered, washed with cyclohexane (50 ml) and the organic solution is evaporated under reduced pressure. The resulting oil is dissolved in MeOH (150 mL) and the solution is added to a 25% solution of MeONa in MeOH (370 ml) kept at 7°C under stirring under N2 atmosphere. After completion of the addition, the mixture is reacted for 1 hour at 7°C, then poured into a 6.5% HCl solution (1000 ml). The mixture is reacted for 10 minutes, extracted with AcOEt, the organic phase is washed with brine, dried over Na2SO4, filtered and evaporated under reduced pressure. The resulting oil is dissolved in toluene, extracted with a 5% K2CO3 solution in water and the aqueous phase is washed with toluene and treated with 37% HCl. The phases are separated and the aqueous phase is extracted with AcOEt, washed with brine, dried over Na2SO4 and evaporated under reduced pressure. The reaction crude is solubilized in toluene under N2 atmosphere and treated with 1,1'-carbonyldiimidazole (18.8 g, 115.6 mmol, 1.1 equivalents). The mixture is left under stirring at 20°C for 2 hours and then at 5°C for 1 hour. The suspended solid is filtered and the resulting toluene solution is directly used in the subsequent step as described in Example 2.

### Example 4. Synthesis of a compound of formula (IV): (R)-5-(tert-butyldimethylsilyloxy)-7-methoxy-3,7-dioxoheptanoic acid

A solution of the compound (V) prepared as in Example 2 (1.1 g, 2.70 mmol) in AcOEt (30 mL) is kept under stirring under H2 atmosphere in the presence of 10% Pd/C (287 mg, 0.27 mmol) at room temperature for 2 hours. The mixture is filtered through perlite and the solvent is evaporated off under reduced pressure a 30°C. The reaction crude (885 mg) is used without further purification in the subsequent preparation.

MS(ES⁺): m/z 341 [M+Na]⁺.

### Example 5. Synthesis of a compound of formula (II): (R,E)-methyl 3-(tert-butyldimethylsilyloxy)-7-(2-cyclopropyl-4-(-4-fluorophenyl)-quinolin-3-yl)-5-oxohept-6-enoate

The compound (**IV**) obtained as in Example 4 (885 mg, 2.78 mmol) is solubilized in DMF (4 mL) under N2 atmosphere and added with 2-cyclopropyl-4-(4-fluorophenyl)quinoline-3-carbaldehyde (271 mg, 0.93 mmol) and piperidine (46 µL, 0.46 mmol). The mixture is left under magnetic stirring at room temperature for 15 minutes, then heated at 40°C for 10 hours. The solution is diluted with AcOEt and washed with 1 N HCl and brine, dried over Na2SO4, filtered and concentrated under reduced pressure. The reaction crude is purified by flash silica gel chromatography (eluent: EtPet/AcOEt 9:1). 280 mg of a pale yellow oil are obtained in 55% yield.

¹H NMR (400 MHz, CDCl3) δ: 7.93 (d, *J* = 8, 1H); 7.63-7.57 (m, 2H); 7.36-7.29 (m, 2H); 7.19-7.16 (m, 4H); 6.31 (d, *J* = 16, 1H); 4.57-4.54 (m, 1H); 3.63 (s, 3H); 2.74-2.62 (m, 2H); 2.50-2.38 (m, 2H); 2.34-2.30 (m, 1H); 1.38-1.36 (m, 2H); 1.05-1.03 (m, 2H); 0.79 (s, 9H); 0.03 (s, 3H); -0.02 (s, 3H). ¹³C NMR (100 MHz, CDCl3) δ: 196.80; 170.89; 163.40; 160.93; 159.51; 146.96; 145.57; 139.70; 133.72; 131.86; 131.27; 131.21; 129.35; 128.57; 126.63; 125.80; 125.46; 125.25; 115.39; 115.18; 65.47; 51.03; 47.61; 41.95; 25.23; 17.43; 15.89; 10.23; 10.16; -5.20; -5.42. MS(ES⁺): m/z 570 [M+Na]⁺.

### Example 6. Synthesis of a compound of formula (I): (3R,5S,E)-7-(2-cyclopropyl-4-(4-fluorophenyl)quinolin-3-yl)-3,5-dihydroxyhept-6-enoic acid calcium salt (Pitavastatin calcium salt)

A solution of (**II**) prepared as in Example 5 (735 mg, 1.34 mmol) in MeOH (12 mL) is cooled to 0°C and 2 M HCl (1 mL, 2.01 mmol) is added drop by drop. The mixture is left under stirring at 20°C for 4 hours. The solution is diluted with AcOEt and washed with a NaHCO3 saturated solution, dried over Na2SO4, filtered and concentrated under reduced pressure. The reaction crude is purified by flash silica gel chromatography (eluent: EtPet/AcOEt 3:2). The resulting solid is dissolved in anhydrous THF (1.5 mL) and MeOH (1.5 mL). The solution is then added drop by drop to a suspension of NaBH4 (55 mg, 1.45 mmol) and 1 M diethylmethoxyborane in THF (1.04 mL, 1.04 mmol) in anhydrous THF (6 mL) at -78°C. The reaction mixture is kept under stirring at -78°C and after 30 min is treated with a NaHCO3 saturated solution and extracted with AcOEt. The organic phase is washed with water, dried over Na2SO4, filtered and concentrated under reduced pressure. The resulting residue is dissolved in AcOEt (6 mL) and the solution is heated to 50°C. A 35% H2O2 aqueous solution (286 µL, 3.33 mmol) is added and the mixture is left under magnetic stirring a 50°C for 1 hour, then added with brine (6 mL) and left to stand at 50°C for 20 minutes; finally a Na2SO3 aqueous solution (223 mg in 6 ml of H2O) is added and the mixture is kept under stirring a 50°C for a further 5 minutes. The organic phase is washed with water, dried over Na2SO4, filtered and concentrated under reduced pressure. The reaction crude is purified by flash silica gel chromatography (eluent: EtPet/AcOEt 3:2). The resulting yellow solid is dissolved in MeOH (7 mL) and treated with 1 M NaOH. The mixture is kept under stirring at 20°C for 2 hours, then the solvent is evaporated off under reduced pressure at 40°C. The residue is dissolved in H2O and added drop by drop with a CaCl2 aqueous solution (103 mg, 0.93 mmol). The immediately formed white solid is kept under stirring at 20°C for 10 hours. The precipitate is filtered, washed with H2O and dried under reduced pressure at 40°C for 4 hours. 348 mg of Pitavastatin calcium salt are obtained as a white solid.

¹H NMR (400 MHz, DMSO) δ: 7.80 (d, J = 8, 1H); 7.58 (t, J = 8, 1H); 7.36-7.20 (m, 6H); 6.44 (d, J = 16, 1H); 6.03 (bs, 1H); 5.56 (dd, *J₁* = 5, *J₂ =* 16, 1H); 4.86 (bs, 1H); 4.11-4.07 (m, 1H); 3.58-3.56 (s, 1H); 2.04-2.00 (m, 1H); 1.89-1.83 (m, 1H); 1.38-1.33 (m, 1H); 1.19-1.04 (m, 2H); 1.00-0.97 (m, 2H); 0.83-0.77 (m, 2H). ¹³C NMR (100 MHz, DMSO) δ: 178.13; 163.04; 160.59; 160.50; 145.94; 143.68; 142.28; 133.12; 132.19; 131.89; 129.67; 128.81; 128.39; 125.71; 123.10; 115.36; 115.15; 68.85; 65.67; 44.50; 43.79; 15.38; 10.75. MS(ES⁺): m/z 444 [M+Na]⁺.

### Example 7. Synthesis of a compound of formula (IV): (R)-5-(tert-butyldimethylsilyloxy)-7-methoxy-3,7-dioxoheptanoic acid

A solution of compound (**V**) prepared as in Example 2 (46.8 g, 115 mmol) in AcOEt (1.3 L) is kept under stirring under H2 atmosphere in the presence of 10% Pd/C (12.2 g, 5.75 mmol) at room temperature for 3 hours. The mixture is filtered through perlite and the solvent is evaporated off under reduced pressure a 30°C. The reaction crude (36.6 g) is used without further purification in the subsequent preparation.

MS(ES+): m/z 341 [M+Na]⁺.

### Example 8. Synthesis of a compound of formula (II): (R,E)-methyl 3-(tert-butyldimethylsilyloxy)-7-(2-cyclopropyl-4-(-4-fluorophenyl)-quinolin-3-yl)-5-oxohept-6-enoate

The compound (**IV**) obtained as in Example 7 (36.6 g, 115 mmol) is solubilized in toluene (550 mL) under N2 atmosphere and 2-cyclopropyl-4-(4-fluorophenyl)quinoline-3-carbaldehyde (100.5 g, 345 mmol, 3 equivalents), 4 Å molecular sieves (70 g) and ß-alanine (30.7 g, 345 mmoles, 3 equivalents) are added. The mixture is left under mechanical stirring at 40°C for 16 hours. The solution is filtered through perlite and concentrated under reduced pressure. The resulting oil is dissolved in AcOEt, washed with 1 N HCl and brine, dried over Na2SO4, filtered and concentrated under reduced pressure. The reaction crude is added with hexane (430 ml) and the suspension is heated at 40°C for 3 hours, then left to cool at room temperature over 16 hours. The solid is filtered, washed with hexane and the organic solution is evaporated under reduced pressure. The resulting oil is used without further purification in the subsequent preparation.

### Example 9. Synthesis of a compound of formula (IX): (3R,5S,E)-7-(2-cyclopropyl-4-(4-fluorophenyl)quinolin-3-yl)-3,5-dihydroxyhept-6-enoic acid (R)-NEA salt

A solution of (**II**) prepared as in Example 8 (27.9 g, 50.9 mmol) in MeOH (470 mL) is adjusted to 0°C and 2 M HCl (65 mL, 127.3 mmol, 2.5 equivalents) is added drop by drop. The mixture is left under stirring at 20°C for 16 hours. I1 organic solvent is evaporated off under reduced pressure, AcOEt (500 ml) is added and the organic phase is washed with a NaHCO3 saturated solution, with brine, dried over Na2SO4, filtered and concentrated under reduced pressure. The reaction crude is purified by flash silica gel chromatography (eluent: toluene/AcOEt 8:2). The resulting solid (16.3 g, 37.6 mmoles) is dissolved in anhydrous THF (55 mL) and MeOH (55 mL). The solution is then added drop by drop to a suspension of NaBH4 (2.0 g, 52.6 mmol, 1.4 equivalents) and 1 M diethylmethoxyborane in THF (38 mL, 37.6 mmol, 1 equivalent) in anhydrous THF (215 mL) kept at -78°C. The reaction mixture is kept under stirring at -78°C and after 1 hour is treated with a NaHCO3 saturated solution and extracted with AcOEt. The organic phase is washed with water, dried over Na2SO4, filtered and concentrated under reduced pressure. The resulting residue is dissolved in AcOEt (230 mL) and the solution is heated at 50°C. A 35% H2O2 aqueous solution (10.5 ml, 115.8 mmol, 3.08 equivalents) is added and the mixture is left under magnetic stirring a 50°C for 1 hour. After that, the mixture is added with brine (230 mL) and left to stand at 50°C for 30 minutes; finally added with a Na2S2O5 aqueous solution (6.6 g in 120 ml of H2O) and kept under stirring a 50°C for a further 10 minutes. The organic phase is washed with brine, dried over Na2SO4, filtered and concentrated under reduced pressure. The obtained solid is dissolved in MeOH (315 mL) and treated with 1 M NaOH (45 ml, 45 mmoles, 1.2 equivalents). The mixture is kept under stirring at 20°C for 2 hours, then the organic solvent is evaporated off under reduced pressure at 40°C. The resulting aqueous phase is washed with methyl t-butyl ether and AcOEt, treated with 1N HCl to pH = 4.5 and extracted with dichloromethane. The organic solution is dried over Na2SO4, filtered and concentrated under reduced pressure; the residue is dissolved in acetone (40 mL) and this solution is added with a solution obtained dissolving (*R*)-NEA (6.5 mL, 40.5 mmoles, 1.1 equivalents) in acetone (18 mL). After completion of the addition, a white solid precipitates, which is added drop by drop with hexane (50 mL) and the suspension is left under stirring at 20°C for 16 hours, then filtered and the solid is then repeatedly recrystallized from acetone and isopropanol/water. 7.1 g of the title compound are obtained, as a crystalline white solid, having both chemical and optical purity degree of 99.5%.

¹H NMR (300 MHz, CDCl3) δ: 7.97 (d, 1H); 7.95 (d, 1H); 7.82 (d, 1H); 7.76 (d, 1H); 7.64 (d, 1H); 7.61-7.43 (m, 5H); 7.33-7.29 (m, 2H); 7.19-7.08 (m, 4H); 6.55 (d, 1H); 5.54-5.47 (m, 4H); 5.11 (dd, 1H); 4.24-4.14 (m, 1H); 3.82-3.68 (m, 1H); 2.47-2.36 (m, 1H); 1.96 (d, 2H);1.66 (d, 3H); 1.38-1.19 (m, 3H); 1.15-0.97 (m, 3H).

### Example 10. Synthesis of a compound of formula (I): (3R,5S,E)-7-(2-cyclopropyl-4-(4-fluorophenyl)quinolin-3-yl)-3,5-dihydroxyhept-6-enoic acid calcium salt (Pitavastatin calcium salt)

The compound of formula (**IX**) obtained as in Example 9 (7.1 g, 12 mmol) is suspended in a water/AcOEt biphasic mixture (30ml/90 ml). 1 N HCl is added to pH = 4.1. The phases are separated and the aqueous phase is extracted with AcOEt; the combined organic phases are added with water (150 ml) and 30% NaOH to pH = 10. The phases are separated and the aqueous phase is washed with AcOEt and methyl t-butyl ether. The aqueous solution is slowly added drop by drop with a CaCl2*₂H2O aqueous solution (530 mg, 3.6 mmol, 0.6 equivalents). The immediately formed white solid is kept under stirring at 20°C for 16 hours. The precipitate is filtered, washed with H2O and dried under reduced pressure at 40°C for 4 hours. 3.4 g (7.8 mmoles) of Pitavastatin calcium salt are obtained as a white solid.

## Claims

1. Process for the preparation of a compound of formula (**II**), or a salt thereof, 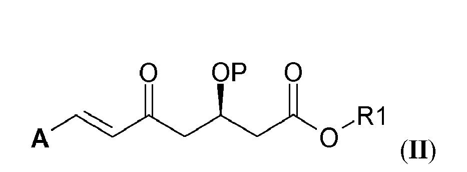 wherein A is a statin residue, P is a protective group, and R₁ is hydrogen, an optionally substituted C₁-C₁₂ alkyl group, cycloalkyl, aryl, or an optionally substituted aryl-C₁-C₁₂ alkyl group;
comprising the condensation of an aldehyde of formula (**III**)
**A**―CHO (III)
wherein A is as defined above, with a β-ketoacid compound of formula (IV), or a salt thereof, 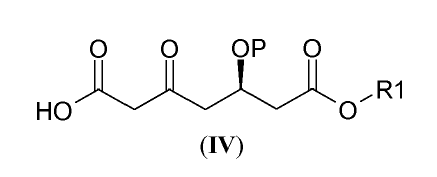 wherein P and R₁ are as defined above, in the presence of a catalyst, if necessary in a solvent, and the subsequent decarboxylation.

2. A process according to claim 1, wherein the catalyst is an organic basic catalyst selected from a secondary amine and a tertiary amine, or a salt thereof, preferably piperidine or a salt thereof.

3. A process according to claim 1, wherein the catalyst is an inorganic basic catalyst selected from a carbonate of an alkali metal and an hydroxide of an alkali metal, preferably sodium or potassium.

4. A process according to claim 1, wherein the catalyst is an α-amino acid, β-amino acid, γ-amino acid, δ-amino acid, or ε-amino acid; wherein said α-amino acid can have a natural or synthetic residue as side chain.

5. A process according to claim 4, wherein the amino acid is chosen from the group comprising glycine, α-alanine, valine, leucine, isoleucine, serine, threonine, phenylalanine, tyrosine, proline, aspartic acid, asparagine, glutamic acid, L,α-aminoadipic acid, β-alanine, γ-aminobutyric acid, δ-aminovaleric acid and ε-aminocapronic acid, taurine and L-phenylserine, preferably β-alanine.

6. A process according to claim 1, wherein the catalyst is an Lewis acid catalyst, preferably selected from ZnCl₂, FeCl₃, TiCl₄, Ti tetraisopropoxide, AlCl₃, etherate BF₃, and a halide or a trifluoromethanesulfonate of a transition metal of the lanthanide series.

7. A process according to claim 1, wherein the solvent is selected from a polar aprotic solvent; acetonitrile; dimethylsulfoxide; an ether; a chlorinated solvent; an ester; an apolar aprotic solvent; and a polar protic solvent; or a mixture of two or more, preferably two or three, of said solvents.

8. A process according to claim 1, further comprising the conversion of a compound of formula (**II**), or a salt thereof, to a statin of formula (**I**), or a salt thereof, 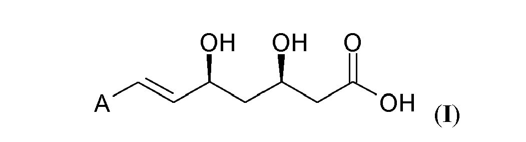 wherein A is a statin residue.

9. A process according to claim 1, wherein a compound of formula (**IV**), or a salt thereof, is obtained by a process comprising the reaction between a compound of formula (**VI**) or a salt thereof, 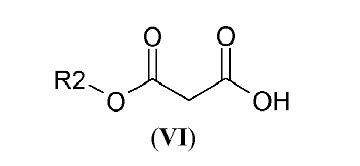 wherein R₂ is an optionally substituted C₁-C₁₂ alkyl group, cycloalkyl, aryl, or optionally substituted aryl-C₁-C₁₂ alkyl;
and a compound of formula (VII), or a salt thereof, 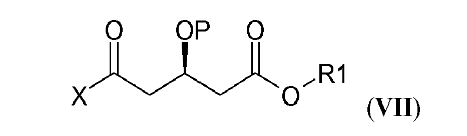 wherein P and R₁ are as defined in claim 1; and X is a leaving group, preferably a halogen, in particular chlorine o imidazole; to obtain a compound of formula (V), or a salt thereof, 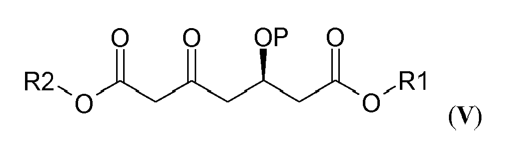 wherein R₁, R₂ and P are as defined above; and the selective removal of the R₂ ester group.

10. Process for the purification of Pitavastatin, or a salt thereof, comprising converting Pitavastatin, or a salt thereof, into a Pitavastatin salt with (R)-naphthylethylamine; recovering it in crystalline form; and converting such salt into Pitavastatin, or a further salt thereof.

11. Process according to claim 10, wherein the Pitavastatin salt in purified form, thus obtained, is the calcium salt.

12. A compound selected from a compound of formula (**IV**) and a compound of formula (**V**), or a salt thereof, 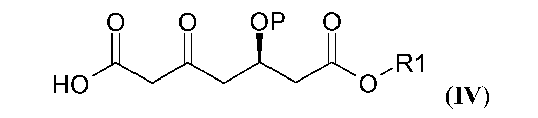 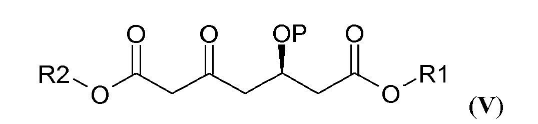 wherein P and R₁ are as defined in claim 1; and R₂ is an optionally substituted C₁-C₁₂ alkyl group, cycloalkyl, aryl, or an optionally substituted aryl-C₁-C₁₂ alkyl.

13. The use of a compound of formula (**IV**), as defined in claim 1, in a process for the preparation of a compound of formula (**II**), as defined in claim 1; or of a statin of formula (**I**), or a salt thereof, 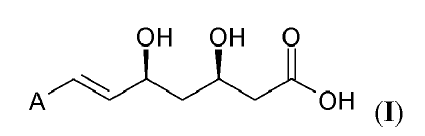 wherein A is a statin residue.

14. Pitavastatin salt with (R)-naphthylethylamine in solid form, preferably in crystalline form, in particular having both a chemical and optical purity equal to or higher than 99%, preferably equal to or higher than 99.5%.

15. A compound of formula (**I**), or a salt thereof, having the following formula 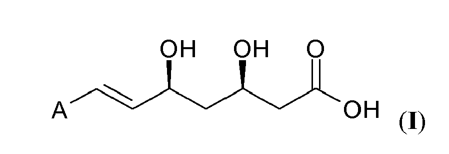 wherein A is a statin residue, having a content in (Z) isomer lower than 1%, preferably ranging from about 0.01 to 0.1%, evaluated by HPLC.
